# EUROPEAN PATENT SPECIFICATION

(11) **EP 4 120 969 B1**
(45) Date of publication and mention of the grant of the patent: **28.02.2024**
(21) Application number: 21726450.6
(22) Date of filing: 18.03.2021
(51) Int. Cl.: A61F 5/441, A61F 5/445

(54) **OSTOMY POUCH INCLUDING MULTI-STAGE FILTER PROTECTION**
STOMABEUTEL MIT MEHRSTUFIGEM FILTERSCHUTZ
POCHE DE STOMIE COMPRENANT UNE PROTECTION DE FILTRE À ÉTAGES MULTIPLES

(30) Priority: 20.03.2020 US 202062992648 P
(43) Date of publication of application: 25.01.2023
(60) Divisional application: 23211546.9
(73) Proprietor: Hollister Incorporated, Libertyville, IL 60048 (US)
(72) Inventor: CZAPLEWSKI, Gregory, J., Libertyville, IL 60048 (US); KUMAR, Kanav, Libertyville, IL 60048 (US)
(74) Representative: FRKelly
(86) International application number: PCT/IB2021/000228
(87) International publication number: WO 2021/186249

(56) References cited:
- US-A- 5 643 234
- US-A- 6 135 986
- US-A1- 2005 070 863

## Description

### BACKGROUND

The following description relates to an ostomy appliance, and more particularly, an ostomy pouch including a deodorizing filter and a multi-stage filter protection. The invention is defined in the claims.

An ostomy bag or pouch includes an inlet configured to receive liquid, semisolid or solid bodily waste discharged from a stoma for collection within the pouch. A known pouch also includes a filter assembly to facilitate odor filtering and egress of gas from the pouch. However, in some instances, liquid, semisolid or solid contents (i.e., bodily waste) may flow to and block the filter assembly, thereby restricting egress of gas through the filter assembly. This may lead to ballooning of the pouch caused by a build-up of gas pressure and undesirable inflation of the pouch.

Disruption to quality of life from the pouch ballooning can be significant for ostomates, for example, anxiety, lack of discretion, fear of leakage, night time considerations, inconvenient user intervention to release gas pressure, etc. Some common methods to release built-up gas include opening a pouch coupling system, which is often referred to as "burping", draining a pouch, and peeling back a skin barrier. Many ostomates have reported spending many hours troubleshooting the pouch ballooning issues and feeling resigned about the current ostomy pouch systems.

US5643234A discloses an ostomy bag including a multi-stage filter system that provides contamination protection for a deodorizing filter in the system. The multi-stage filter system also includes protection elements that are impassable to semi-liquid waste material but permit passage of gaseous waste. The protection elements are located such that gaseous waste must pass through the protection elements before it passes through the deodorizing filter. In one embodiment of the invention, the protection elements include a fluid and gas-impermeable cover layer with fluid bypass ports. The fluid and gas-impermeable cover layer is followed by a barrier filter that restricts passage of semi-liquid waste material. The barrier filter is followed by a liquid-impermeable membrane that prevents passage of liquid. The liquid-impermeable membrane is followed by a moisture-absorbent pad that is adjacent the deodorizing filter. The moisture-absorbent pad absorbs moisture condensate that may progress as vapor through the liquid-impermeable membrane. Thus semi-liquid waste cannot contact the deodorizing filter, since it cannot bypass the protection elements to reach the deodorizing filter.

US6135986A discloses an ostomy appliance comprising a front wall and a rear wall of flexible material, the rear wall having an opening into the bag by which waste material can enter the bag and one of the walls has one or more vents through which gas may escape from the bag and having a filter covering said vent, said filter comprising an elongated, substantially flat filter body of a porous filter material interposed between gas and liquid impervious walls which are sealed to the body along its longitudinal side edges; gas inlet and outlet openings being provided in communication with the filter material adjacent to its respective longitudinal end regions, wherein both of the gas and liquid impervious walls are sealed to the upper end lower surfaces of the filter body, the arrangement being so that in use gas flows longitudinally through the filter from the inlet opening to the outlet opening, such gas flow being by a hydrophobic sheet, wherein the inlet opening is covered with a microporous oleophobic membrane and wherein a foam material is placed between the front wall and the rear wall and covering the inlet opening of the vent shows improved resistance against wetting and blocking of the filter.

US2005070863A1 discloses an ostomy appliance comprising a front wall and a rear wall of flexible material forming a bag, the rear wall having an opening into the bag by which waste material can enter the bag and one of the walls has one or more vents through which gas may escape from the bag and having a filter covering said vent wherein gas inlet and outlet openings and provided in communication with a filter body, the arrangement being so that in use gas flows through the filter from the inlet opening to the outlet opening, such gas flow being confined to said filter body, wherein the gas inlet opening to the filter is provided with at least two separate and independent closed pathways having separated inlet openings communicating with the bag and separate and independent outlet openings communicating with the inlet opening of the filter, and wherein each of the outlet openings of the separate pathways are covered by a microporous membrane shows improved resistance against blocking of the inlet opening of filter.

Accordingly, it is desirable to provide an improved ostomy pouch system that can minimize ballooning while still providing comparable or better odor filtration.

### SUMMARY

The present disclosure provides an ostomy pouch as detailed in claim 1. Advantageous features are provided in dependent claims.

In one aspect, an ostomy pouch including a filter assembly and a multi stage filter protection is provided. The ostomy pouch may include a body-side wall and a distal- side wall
joined at an outer periphery and defining an interior volume comprising a collection area. The ostomy pouch may further comprise an inlet for receiving ostomy effluent and an outlet for egress of gas collected in the collection area. The filter assembly may be arranged to cover the outlet. The multi-stage filter protection may be configured to protect the filter assembly and may comprises a prefilter and a protective panel covering the prefilter.

In an embodiment, the filter assembly may be attached to an outer surface of one of the body-side wall and the distal-side wall, and the multi-stage protection may be arranged inside the pouch. For example, the filter assembly may be attached to an outer surface of the distal-side wall and the prefilter and the protective panel may be attached to an inner surface of the distal-side wall. In other embodiments, the filter assembly and the multi-stage protection may be arranged inside the pouch.

The protective panel may be formed from a microperforated film, an embossed film, or a microperforated embossed film. In an embodiment, the protective panel may be formed from a microperforated film comprising a plurality of openings having a diameter of about 100 µm to 500 µm, wherein the plurality of openings may be arranged to provide a pore-density of about 25 ppi to about 300 ppi. The protective panel may be sealed to the distal-side wall and/or to the body-side wall along an outer peripheral seal. The plurality of openings may be provided in a portion of the protective panel or throughout the entire surface of the protective panel. For example, the plurality of openings may be provided in a lower portion or an upper portion of the protective panel.

In an embodiment, the protective panel may be formed from a microperforated film including a first set of microperforations comprising a plurality of openings having a diameter of about 300 µm to about 500 µm and a second set of microperforations comprising a plurality of openings having a diameter of about 50 µm to about 200 µm, wherein the first set of microperforations may be arranged proximate a lower periphery of the protective panel and the second set of microperforations may be arranged above the first set of microperforations. In such an embodiment, the first set of microperforations may be configured to allow ostomy effluent accumulated between the protective panel and the pouch wall to flow down to the collection area. The second set of microperforations may be provided in at least about 30% of the protective panel area at a pore-density of about 100 ppi to about 200 ppi to allow gas to flow through the protective panel even after some of the microperforations are blocked by ostomy effluent.

The protective panel may be sealed to the distal-side wall along a lower periphery to provide a horizontal seal. A portion of the protective panel proximate the filter assembly may be free of microperforations. The horizontal seal may be a discontinuous heat seal or the protective panel may include at least one slit or opening proximate the lower periphery configured to allow liquid accumulated between the protective panel and the distal-side wall to flow down into the collection area.

In an embodiment, the prefilter may include a first layer formed from a reticulated foam or an open cell foam. For example, the first layer may be formed from a reticulated polyurethane (PU) foam. In some embodiments, the prefilter may also include a second layer. In such embodiments, the first layer may be laminated to the second layer, wherein the prefilter may be attached to the distal-wall by heat sealing the second layer to an inner surface of the distal-wall. The second layer may be formed from a polyester nonwoven or a spunbond-meltblown-spunbond polypropylene (SMS PP) nonwoven.

In an embodiment, the filter assembly may comprise a membrane layer, a backing layer, and a filter media arranged therebetween. The filter assembly may be attached to an outer surface or of the one of the body-side wall and the distal-side wall or an inner surface of the one of the body-side wall and the distal-side wall, such that the membrane layer covers the outlet. The backing layer may be formed from a low density polyethylene film. The filter media may be formed from an activated carbon impregnated foam, which may be hydrophobic. In an embodiment, the filter media may be formed from an activated carbon reticulated PU foam. The membrane layer may be formed from a SMS PP nonwoven. In an embodiment, the SMS PP nonwoven may have a basis weight of about 40 gsm to about 80 gsm.

In an embodiment, the filter assembly may be configured to provide a radial gas flow path through the filter media. In such an embodiment, the filter assembly may be configured to direct the gas egressing through the pouch outlet to flow through the membrane layer and radially flow through the filter media and exit the filter assembly through at least one gas outlet provided proximate an outer periphery of the filter assembly.

In an embodiment, the filter assembly and the multi-stage protection may be configured and arranged to allow the gas collected in the collection area to flow through microperforations of the protective panel and flow through the prefilter and exit the ostomy pouch through the outlet and flow through the membrane layer and filtered via the filter media before exiting the filter assembly.

In another embodiment, the filter assembly and the multi-stage protection may be configured and arranged to allow the gas collected in the collection area to flow through microperforations of the protective panel, flow through the prefilter, and flow through the filter media radially, and flow through the membrane layer before exiting the ostomy pouch through the outlet.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a perspective view of an ostomy appliance comprising a filter assembly and a multi-stage filter protection according to an embodiment;
FIG. 2 is a schematic cross-sectional illustration of a foam prefilter arranged adjacent a filter assembly according to an embodiment;
FIG. 3 is an illustration of a microperforated protective panel according to an embodiment;
FIG. 4 is an illustration of an embossed protective panel comprising a diamond-shaped pattern according to an embodiment;
FIG. 5 is a perspective view of an ostomy pouch comprising a filter assembly and a multi-stage filter protection according to an embodiment;
FIG. 6 is a schematic illustration of a filter assembly and a multi-stage filter protection according to another embodiment;
FIG. 7 is a schematic illustration of a filter assembly with a multi-stage filter protection according to yet another embodiment;
FIG. 8 is a partial exploded view of an ostomy pouch comprising a filter assembly and a multi-stage filter protection according to an embodiment;
FIG. 9 is a microscopic image of a reticulated foam according to an embodiment;
FIG. 10 is a microscopic image of a reticulated foam filled with activated carbon according to an embodiment;
FIG. 11 is an illustration of an ostomy pouch mounted on a test fixture for an airflow rate test according to an embodiment;
FIG. 12 is an illustration of a liquid hold-out test set up according to an embodiment;
FIG. 13 is an illustration of an ostomy filter clamped in a test fixtured in the liquid hold-out test set up of FIG. 12;
FIG. 14 is a schematic perspective view of an ostomy pouch including a protective panel formed from a perforated film according to an embodiment;
FIGS. 15A-15D are schematic illustrations of perforations according to various embodiments;
FIG. 16 is a graph of volatile analysis test results using H₂S challenge gas;
FIG. 17 is a graph of volatile analysis test results using methyl mercaptan challenge gas; and
FIG. 18 is a partial exploded view of an ostomy pouch comprising a filter assembly and a multi-stage filter protection according to another embodiment.

### DETAILED DESCRIPTION

One inch equals 2,54 cm.

One pound equals 0,45 kilogram.

One pound per square inch (psi) equals 0,07 kilogram per square centimeter.

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

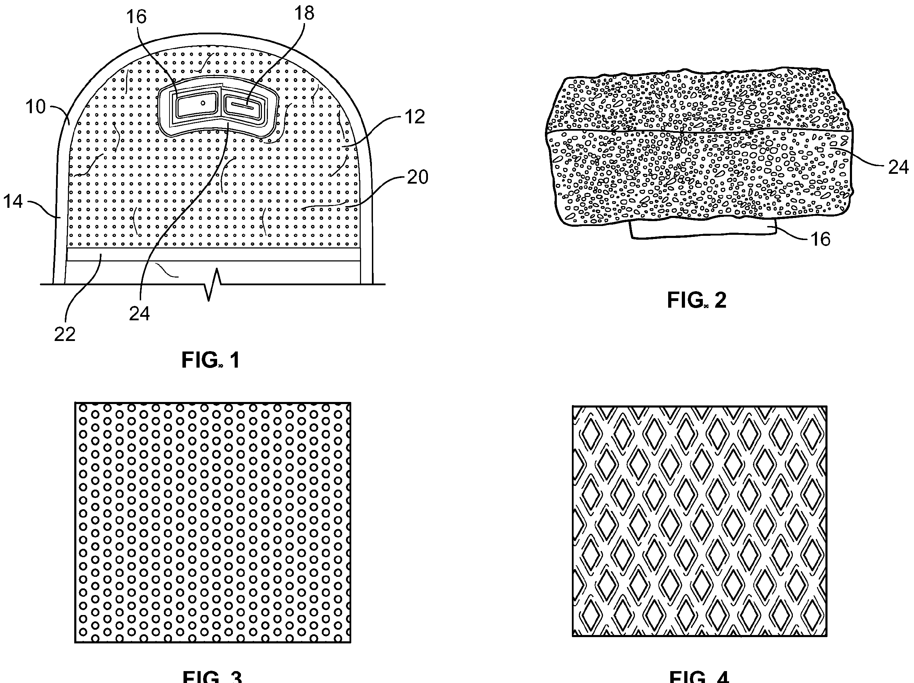

FIG. 1 shows an ostomy appliance 10 according to an embodiment. The ostomy appliance 10 may be an ostomy pouch. The ostomy appliance 10 may include outer walls 12 comprising a body-side wall and a distal-side wall joined together at an outer periphery 14. The outer walls 12 may define an interior volume, which according to one embodiment, may include a collection area. The ostomy appliance 10 may also include a filter assembly 16 attached to one of the outer walls 12 over a gas outlet provided therein. The filter assembly 16 may be fluidically connected to the collection area to allow gas to flow through the filter assembly 16 and exit through the gas outlet. The filter assembly 16 may include a filter media 18 comprising a charcoal, carbon or other suitable deodorizing material for deodorizing gas passing therethrough.

The ostomy appliance 10 may include a multi-stage protection to reduce a risk of ostomy effluent clogging the filter assembly 16 and cause ballooning of the ostomy pouch. In an embodiment, the ostomy appliance 10 may include a two-stage protection comprising a protective panel 20 and a prefilter 24. A first stage protection may be the protective panel 20 arranged between two opposing outer walls 12. In the embodiment of FIG. 1, the filter assembly 16 may be attached to an upper portion of the distal-side wall. In this embodiment, the protective panel 20 may be arranged in an upper portion of the ostomy appliance 10 and sealed to the opposing outer walls 12 along the peripheral seal 14. The protective panel 20 may also be sealed to the distal-side wall along a lower periphery providing a horizontal seal 22.

The protective panel 20 may be formed from a perforated film configured to block ostomy effluent while allowing gas to flow therethrough. In an embodiment, the protective panel 20 may comprise a plurality of microperforations having a micro unit sized perforations as shown in FIG. 3. In some embodiments, the protective panel 20 may comprise perforations of various sizes and/or perforations in various patterns. For example, the protective panel 20 may comprise microperforations in a lower portion and may be free of perforation in an upper portion proximate the filter assembly 16.

In another embodiment, the protective panel 20 may be formed from a textured film and arranged to protect the filter assembly 16. For example, the protective panel 20 may be formed from an embossed film configured to block ostomy effluent while allowing gas flow through. The embossed film may also be configured to minimize pancaking of the ostomy bag, wherein the protective panel 20 is arranged to prevent the outer walls 12 from stick together and to facilitate ostomy effluent to drop to the bottom of the ostomy pouch. The protective panel 20 may be embossed in various patterns, various depths, and/or at various locations. In an embodiment, the protective panel 20 may be embossed in a diamond-shaped pattern as shown in FIG. 4. The diamond-shaped pattern may be configured to define diagonal channels providing gas paths to the filter assembly 16. The diagonal channels may be configured to reduce a risk of the gas paths being blocked by ostomy effluent. Such diagonal channels may be less likely to be blocked than straight vertical and horizontal channels, such as those defined in a checkerboard pattern. In an embodiment, the protective panel 20 may be formed from a textured film that is free of textures in at least a portion of the textured film proximate a prefilter 24. The protective panel 20 may include a gas inlet opening or may be attached to the outer wall 12 discontinuously to provide a gas flow path.

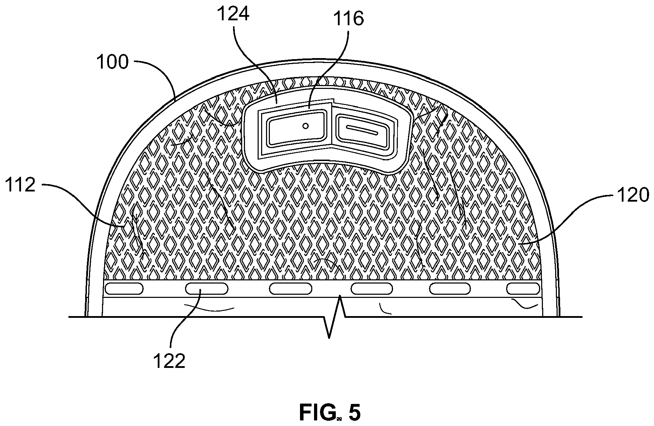

FIG. 5 is a partial perspective view of an ostomy pouch 100 according to an embodiment. The ostomy pouch 100 may be configured similar to the ostomy pouch 10 generally comprising a filter assembly 116 and a two-stage protection including a protective panel 120 and a prefilter 124. In this embodiment, the protective panel 120 may be formed from an embossed film comprising a diamond shaped pattern, which may be sealed to a pouch wall 112 via a discontinuous heat seal 122 to provide a gas flow path.

In an embodiment, the protective panel 20 may be formed from an embossed and perforated film. For example, the protective panel 20 may be formed from an embossed film comprising a plurality to microperforations. In such an embodiment, the protective panel 20 may be free of textures and perforations proximate the prefilter 24.

In some embodiments, the protective panel 20 may include at least one slit or cut in a lower portion to allow any ostomy effluent accumulated between the protective panel 20 and the outer wall 12 to flow down to the collection area. In an embodiment, the protective panel 20 may include a first set of microperforations arrange proximate a lower periphery of the protective panel 20 and a second set of microperforations arranged thereabove, wherein the first set of microperforations have a diameter larger than that of the second set of microperforations. In such an embodiment, the first set of microperforations may be configured to allow ostomy effluent accumulated between the protective panel 20 and the outer wall 12 to flow down to the collection area.

A second stage filter protection may be provided by the prefilter 24. FIG. 2 is a schematic cross-sectional illustration of the prefilter 24 provided adjacent the filter assembly 16 (the thickness of the prefilter 24 is exaggerated). The prefilter may be formed from a suitable material configured to restrict ostomy effluent including thinner (lower viscosity) effluent while allowing gas to flow therethrough. Suitable prefilter materials include, but are not limited to, foams, such as a reticulated open cell foam, textiles, microfleece, nonwoven, and the like. Some of the suitable prefilter materials, such as a polyurethane foam, textile, and microfleece may be a hydrophobic material that may repel liquid ostomy effluent away from the prefilter 24.

In an embodiment, the prefilter 24 may be formed from a reticulated polyurethane foam. The foam may have a pore size of about 10 pores per inch (ppi) to about 250 ppi, preferably about 30 ppi to about 200 ppi. For example, the foam may have a pore size of about 40 ppi to about 60 ppi. In the embodiment of FIGS. 1 and 2, the prefilter 24 may be sized and arranged to approximately cover the filter assembly 16. In another embodiment, the prefilter 24 may be sized substantially larger than the filter assembly 16. In yet another embodiment, the prefilter 24 may be arranged to substantially fill a compartment defined between the protective panel 20 and the distal-side wall. The prefilter 24 may be relatively thin to minimize the bulkiness of the ostomy appliance 10. In an embodiment, the prefilter 24 may be formed from an open-cell foam having a thickness of about 1/32 inches to about 1/2 inches, preferably about 1/16 inches to about 1/4 inches, and more preferably about 1/8 inches. The prefilter 24 may be configured such that a user may squeeze out liquid absorbed therein by applying pressure through pouch outer walls.

In some embodiments, the ostomy appliance 10 may include more than two stages of filter protection. For example, the ostomy appliance 10 may include at least one heat seal, which may be configured and arranged to protect the filter assembly 16. In an embodiment, the ostomy appliance 10 may include at least one heat seal that seals the protective panel 20 to one of the outer walls 12 proximately below the filter assembly 16 or surrounding a lower portion of the filer assembly 16 to deter ostomy effluent from reaching the filter assembly 16 and/or the prefilter 24.

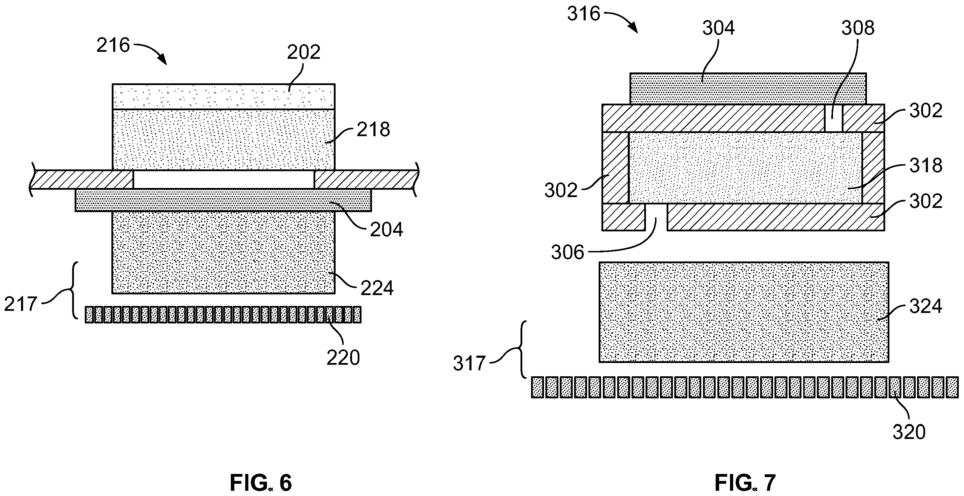

FIG. 6 is a schematic illustration of a filter assembly 216 and a multi-stage filter protection 217 according to an embodiment. In this embodiment, the filter assembly 216 may comprise a backing layer 202 and an activated carbon foam filter media 218. The multi-stage filter protection 217 may comprise a membrane layer 204, a prefilter 224 formed from a foam material, and a protective panel 220 formed from a microperforated film. The membrane layer 204, which may be formed from a suitable gas permeable material, may be arranged between the filter media 218 and the prefilter 224. The suitable gas permeable material for the membrane layer 204 may include, but are not limited to, membrane materials, gas permeable polymeric films, nonwovens, and the like.

FIG. 7 is a schematic illustration of a filter assembly 316 and a multi-stage filter protection 317 according to another embodiment. In this embodiment, the filter assembly 316 may comprise a membrane layer 304 and an activated carbon polyester (PET) filter media 318 enclosed in a packet film 302 formed from a gas impermeable material, wherein a gas inlet opening 306 and a gas outlet opening 308 are provided in the packet film 302. The multi-stage filter protection 317 may comprise a foam prefilter 324 and a microperforated film protective panel 320.

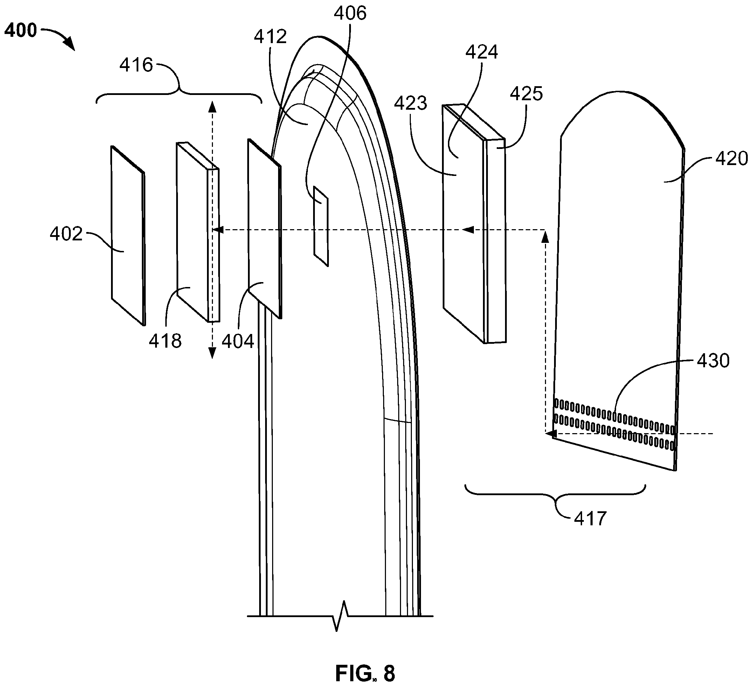

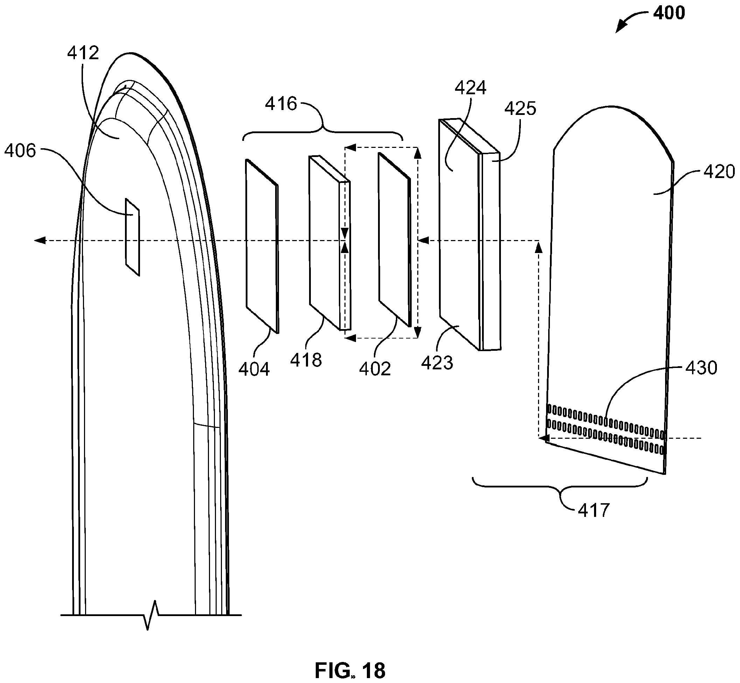

FIG. 8 is a partial exploded view of an ostomy pouch 400 according to an embodiment. The ostomy pouch 400 may be configured similar to the ostomy pouch 10 generally comprising a filter assembly 416 and a multi-stage protection 417 including a protective panel 420 and a prefilter 424. In this embodiment, the filter assembly 416 may be attached to an outer surface of a pouch wall 412 while the multi-stage protection 417 may be arranged inside the pouch. In another embodiment, the filter assembly 416 and the multi-stage protection 417 may be arranged inside the pouch as shown in FIG. 18.

The filter assembly 416 may comprise a backing layer 402, a filter media 418, and a membrane layer 404. The filter assembly 416 may be arranged to cover a gas outlet opening 406 defined in the pouch wall 412 and attached to the pouch wall 412, for example via heat sealing. The membrane layer 404 may be formed from a suitable gas permeable material configured to allow gas to flow therethrough while providing protection against ostomy effluent. The filter media 418 may be formed from a suitable filter material configured to deodorize ostomy gas. The backing layer 402 may be formed from a suitable material that has a relatively low gas permeability or gas impermeable and configured to direct gas to flow radially through the filter assembly 416. In this embodiment, gas collected in the ostomy pouch 400 may egress through the outlet opening 406 and flow into the filter assembly 416 through the membrane layer 404 and radially flow through the filter media 418 before exiting the filter assembly 416 as indicated by arrows in FIG. 8. The filter assembly 416 may comprise at least one gas outlet proximate a periphery of the filter assembly 416. For example, the filter assembly 416 may comprise a gas outlet defined by an unsealed periphery.

The radial gas flow length through the filter media 418 may be affected by the size of the outlet opening 406 and the size of the filter media (larger the outlet opening 406, shorter the gas path through the filter media 418). Further, the flow rate of gas egressing the ostomy pouch 100 through the filter assembly 116 may be adjusted by configuring the size of the outlet opening 106 and gas flow properties of the membrane layer 104, such as a porosity of the membrane layer 104. In an embodiment, the size of the outlet opening 406 and the membrane layer 404 may be configured to allow sufficient gas egress to minimize pouch ballooning while still providing a gas flow length through the filter media 418 for excellent odor deodorization.

In an embodiment, the outlet opening 406 may have an area of about 0.02 inch² to about 0.15 inch², preferably about 0.05 inch² to about 0.08 inch², and more preferably 0.06 inch² to about 0.07 inch².
The outlet opening 406 may be provided in various shapes, for example, circular opening, elliptical opening, rectangular opening, square opening, etc. In an embodiment the outlet opening 406 may be defined by a square shaped opening having an area of about 0.0625 inch² (0.25 inches x 0.25 inches).

Suitable materials for the backing layer 402 may include, but are not limited to, polymeric films having a substantially lower gas permeability compared to the filter media 418. For example, the backing layer 402 may be formed from a polymeric film, such as a low density polyethylene (LDPE) film. The backing layer 402 may have a thickness of about 2 mil to about 10 mil, preferably about 3 mil to about 7 mil, and more preferably about 5 mil.

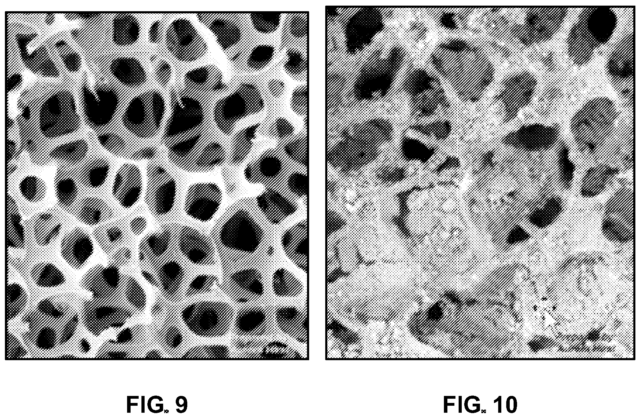

The filter media 418 may be formed from a suitable filter material comprising charcoal, carbon or other suitable deodorizing materials for deodorizing gas. Suitable filter materials for the filter media 418 may include, but are not limited to, activated carbon foam materials, such as a filter material comprising a reticulated foam and activated carbon, activated carbon nonwoven, and activated carbon cloth. FIG. 9 is a microscopic image of a reticulated foam according to an embodiment, and FIG. 10 is a microscopic image of a reticulated foam filled with activated carbon according to an embodiment. The filter media 418 may have a thickness of about 0.03 inches to about 0.15 inches, preferably about 0.06 inches to about 0.12 inches, and more preferably about 0.07 inches to about 0.1 inches.

In an embodiment, the filter media 418 may be formed from a reticulated polyurethane (PU) foam comprising activated carbon and having a thickness of about 0.089 inches, such as PU foam filter materials available from Freudenberg. Such a PU foam filter material may be hydrophobic and may provide additional advantages for the filter assembly 416 arranged on an outer surface of the pouch. For example, the hydrophobic filter media 418 may resist water and eliminate a need for a filter sticker when the filter assembly 416 is exposed to water, for example during shower or swimming.

The membrane layer 404 may be formed from a suitable gas permeable material. Suitable gas permeable materials for the membrane layer 404 may include, but are not limited to, ePTFE (expanded polytetrafluoroethylene) membrane, UHMW PE (ultra high molecular weight polyethylene) membrane, pulp/polyester membrane, spunmelt PP (polypropylene) membrane, SMS PP (spunbond meltblown spunbond polypropylene) nonwoven, and the like. The membrane layer 404 may have a thickness of about 0.5 mil to about 15 mil, preferably about 0.8 mil to about 12 mil. In an embodiment, the membrane layer 404 may be formed from a tri-laminate SMS PP nonwoven comprising a spunbond PP top layer, a meltblown PP middle layer, and a spunbond PP bottom layer having a basis weight of about 10 gsm to about 500 gsm, preferably about 30 gsm to about 120 gsm, and more preferably about 40 gsm to about 80 gsm. For example, the membrane layer 404 may be formed from a SMS PP nonwoven having a basis weight of about 44 gsm available under Style T063-73960 from Precision Fabrics Group Inc. In another embodiment, the membrane layer 104 may be formed from a microporous UHMW PE membrane having a basis weight of about 1 gsm to about 20 gsm, preferably about 2 gsm to about 5 gsm, a thickness of about 10 µm to about 50 µm, preferably about 15 µm to about 40 µm, and a porosity of about 60% to about 90%, preferably about 70% to about 85%. For example, the membrane layer 104 may be formed from a microporous UHMW PE membrane having a basis weight of about 3 gsm, a thickness of about 20 µm, and a porosity of about 83%, which is available under the tradename Solupor^{®} membranes 3P07A from Lydall Performance Materials B.V.

In an embodiment, the filter assembly 416 may be configured to minimize ballooning while still providing excellent odor filtration and preventing ostomy effluent leakage. Such properties of a filter assembly may be evaluated by analyzing airflow rate through the filter assembly, liquid hold-out, which measures a pressure at which a liquid is forced through a membrane layer of the filter assembly, and deodorization data.

In the embodiment of FIG. 8, the airflow rate and liquid hold-out of the filter assembly 416 may be mainly determined by the properties of the membrane layer 404. The cost of the membrane layer for many prior art filter assemblies, for example, those including a membrane layer formed from an ePTFE membrane or UHMW PE membrane, is often the largest portion of the total material cost of the filter assembly. For example, the cost of the membrane layer formed from an ePTFE membrane may make up over 50% of the total material cost of a filter assembly.

The inventors of the present application have researched and analyzed numerous different membrane materials, nonwoven materials, fabric materials, and other gas permeable materials to identify a suitable material for a filter membrane layer that can provide comparable or better filter properties at a substantial cost saving. After substantial time and investment in research and development, it was discovered that a filter assembly comprising a membrane layer formed from a SMS PP nonwoven material, which is typically used for hospital gowns, may provide surprisingly excellent filter membrane properties, such as airflow rate and liquid hold-out, at a substantially lower cost. For example, the cost of a SMS PP nonwoven material can be as low as about 1% of the cost of an ePTFE membrane material. Airflow rate and liquid hold-out data for various membrane materials are shown in Table 1.

**Table 1 Airflow Rate and Liquid Hold-Out Data of Filter Membranes**

| **Membrane** | **Airflow rate @0.18psi (cc/s)** | **Liquid (H₂O) hold-out (psi)** |
|---|---|---|
| SMS PP nonwoven (T063-73960, PFG) | 28.6 (filter assembly) | 1.32 (membrane only) |
| Spunmelt PP | 22.4 (filter assembly) | 0.7 (membrane only) |
| Pulp/polyester | 20.9 (filter assembly) | 0.27 (filter assembly) |
| UHMW PE (Solupor^{®} 3P07A, Lydall) | 8.06 (membrane only) | 4 (membrane only) |
| e-PTFE | 11.2 (filter assembly) | 6 (filter assembly) |
| e-PTFE | 7.28 (filter assembly) | 6 (filter assembly) |
| UHMW PE | 4.1 (filter assembly) | >10 (filter assembly) |

The airflow rate was tested using Isaac HD Multi-Function Leak Tester (Isaac tester) equipped with a Mass Flow Meter (MFM), which measures a mass flow rate of air through a pouch to maintain a specified pressure. A square shaped and Teflon coated test plate including alignment holes near each of the corners and an opening in the center to allow air to pass into a pouch was used to mount a pouch. A test fixture including two air cylinders was used to clamp the test plate and the pouch mounted thereto. The test fixture included a hole defined therein to allow air to pass from a pressure transducer into the pouch.

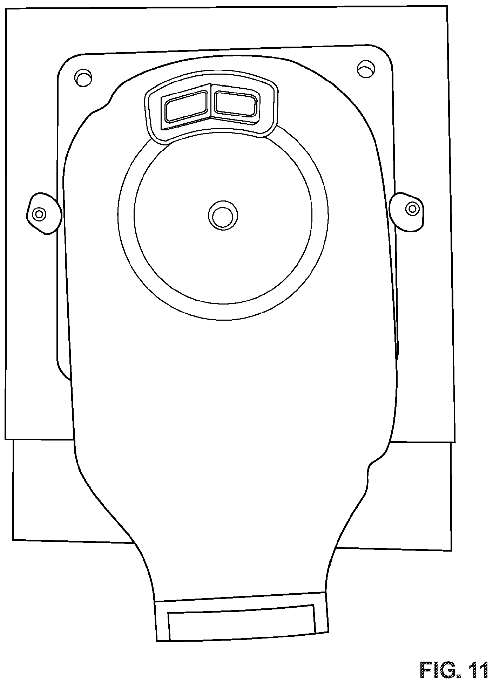

The airflow rate data in Table 1 were collected by measuring an air flow rate to maintain a 0.18 psi pressure in a sample pouch including a filter assembly or a membrane (as indicated in Table 1) attached thereto to cover a gas outlet opening. The sample pouch was attached to the test plate by removing a barrier backing and centering a pouch starter hole over the center hole of the test plate, such that no air channels are formed between the barrier and the test plate. The test plate with the pouch mounted thereto is attached to the test fixture using the locating pins to guide alignment and pneumatically clamped as shown in FIG. 11. Using the Isaac tester, an air flow rate to maintain a pressure of 0.18±0.018 psi was measured and recorded.

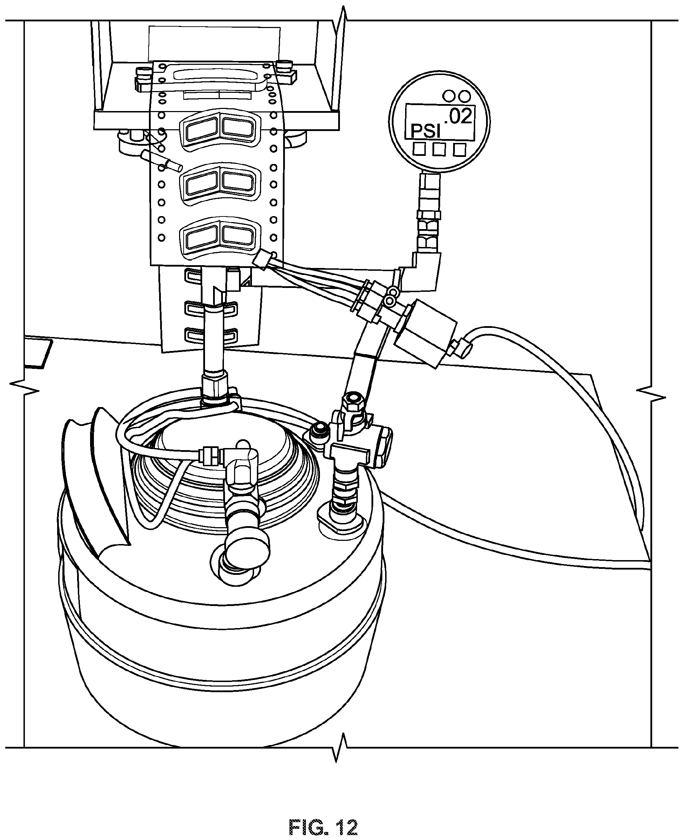

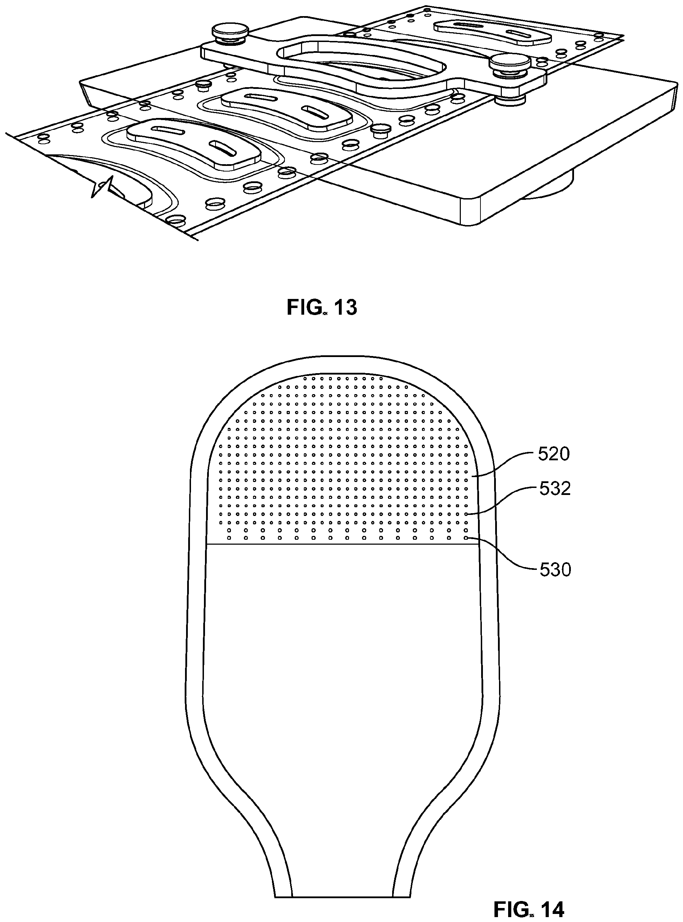

The liquid hold-out was tested using a test equipment system including a liquid pressure tank, an air source, and a liquid pressure gauge (FIG. 12) to measure a pressure at which a liquid (water was used for the data provided in Table 1) is forced through a membrane or a membrane layer of a filter assembly. A sample membrane or a sample filter assembly (as indicated in Table 1) was placed on a fixture and a filter clamp was positioned over the fixture, such that the filter clamp is aligned over the membrane or filter assembly as shown in FIG. 13. After closing the filter clamp via a pneumatic valve, the system pressure was raised until water penetrated the sample membrane or the membrane layer of the sample filter assembly.

After detailed examination and careful studies of ostomy pouch ballooning phenomenon, effluent leakage through ostomy filters, and filtering of ostomy gas, and analyses of airflow rate and liquid hold out data of numerous membrane materials and filter assemblies, it was discovered that an ostomy pouch comprising a filter assembly configured to have an airflow rate @ 0.18 psi of greater than about 10 cc/s and less than about 40 cc/ss and a liquid (water) hold-out of greater than about 0.9 psi may minimize pouch ballooning while still preventing ostomy effluent leakage.

In an embodiment, the filter assembly 416 may comprise the backing layer 402 formed from a LDPE film having a thickness of about 5 mil, the filter media 418 formed from an activated carbon reticulated PU foam having a net density of about 26 kg/m³ to about 30 kg/m³ (tested according to ISO 845), and the membrane layer 404 formed from a SMS PP nonwoven having a basis weight of about 44 gsm, wherein the filter assembly 416 may be covering the outlet opening 406 having an area of about 0.06 inch² to about 0.07 inch² and configured to have an airflow rate @0.18 psi of greater than about 10 cc/s and less than about 40cc/sand a liquid (water) hold-out of greater than about 0.9 psi and less than about 3.0 psi. In an embodiment, the filter assembly 416 may be configured to cover the outlet opening 406 having an area of about 0.0625 inch² and have an airflow rate @0.18 psi of greater than about 15 cc/s and less than about 35 cc/s and a liquid (water) hold-out of greater than about 1.0 psi and less than about 2.0 psi. The filter assembly 416 may be provided in various shapes, for example, circular, elliptical, rectangular, or square shapes.

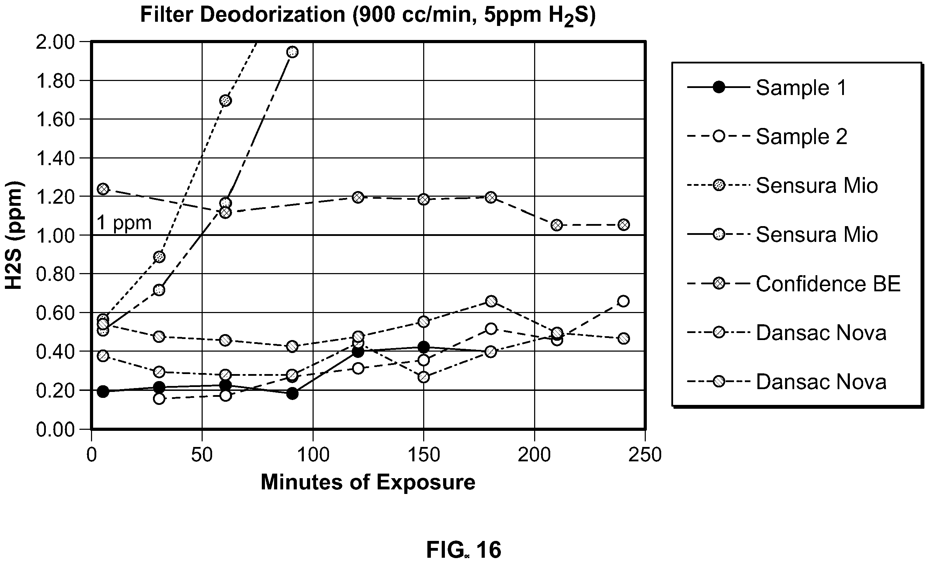

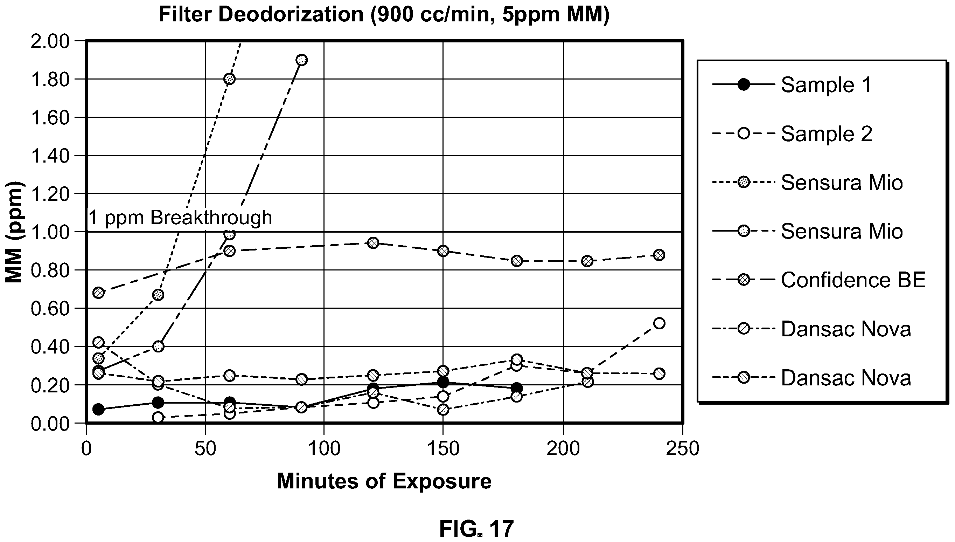

Samples of the filter assembly 416 having a square-shaped body with the side length of 1.165 inches and comprising the backing layer 402 formed from a LDPE film having a thickness of about 5 mil, the filter media 418 formed from an activated carbon reticulated PU foam having a net density of about 26 kg/m³ to about 30 kg/m³ (tested according to ISO 845), and the membrane layer 404 formed from a SMS PP nonwoven having a basis weight of about 44 gsm were prepared and tested for deodorization properties along with prior art filter assemblies. Volatile analyses using a challenge gas containing 5ppm H₂S in dry nitrogen and a challenge gas containing 5ppm methyl mercaptan (MM) in dry nitrogen were conducted. The test parameters included: challenge gas humidified to 25% RH (relative humidity), a flow rate of challenge gas to filter of 15 cc/s, and a back pressure of 0.8 psi. FIG. 16 is a graph of volatile analysis test results using the H₂S challenge gas, and FIG. 17 is a graph of volatile analysis test results using the MM challenge gas. As shown in FIGS. 16 and 17, the samples of the filter assembly 416 (referred to as "Sample 1" and "Sample 2") exhibited better deodorization properties when compared to Coloplast SenSura^{®} Mio filter assembly samples including an e-PTFE membrane and Salts Healthcare Confidence BE^{®} filter assembly samples including an e-PTFE membrane, and exhibited similar deodorization properties when compared to Dansac NovaLife filter assembly samples including a UHMW PE membrane.

Referring back to FIG. 8, the multi-stage filter protection 417 may comprise the prefilter 424 arranged to cover the gas outlet opening 406 and sealed to an inner surface of the pouch wall 412 and the protective panel 420 covering the prefilter 424 and sealed to an inner surface of the pouch wall 412. In such an embodiment, the protective panel 420 may function as a coarse prefilter and a first line of protection and the prefilter 424 may function as a fine prefilter and a second line of protection to provide a multiple protection for the filter assembly 416 from ostomy effluent collected in the pouch.

In an embodiment, the prefilter 424 may comprise a first layer 425 and an optional second layer 423. The first layer 425 may be configured for fine particulate blocking and formed from any suitable material comprising sufficient gas flow path/channels to provide a substantially lower gas flow resistance when compared to the optional second layer 423 or the membrane layer 404. Suitable materials for the first layer 425 may include, but are not limited to, open-cell foams and reticulated foams including about 10 ppi to about 250 ppi, preferably about 30 ppi to about 200 ppi. For example, the first layer 425 may be formed a reticulated foam including about 200 ppi. Suitable materials for the first layer 425 are not limited to foam materials and may include other similar materials configured for fine particular blocking and a relatively low gas flow resistance. The first layer 425 may have a thickness of about 1/32 inches to about 1/2 inches, preferably about 1/16 inches to about 1/4 inches, and more preferably about 1/8 inches. In an embodiment, the first layer 425 may be formed from, a reticulated PU foam having about 45 ppi and a thickness of about 1/8 inches. In some embodiment, the first layer 425 may be laminated to the second layer 423.

The second layer 423 may be formed from a suitable material configured to provide some support for the first layer 425 during handling and processing and heat sealability to the pouch wall 412. Suitable materials for the second layer 423 include, but are not limited to, nonwoven materials, membrane materials, gas permeable polymeric materials and the like. For example, the second layer 423 may be formed from a polyester (PET) nonwoven or a SMS PP nonwoven having a basis weight of about 10 gsm to about 500 gsm, preferably about 20 gsm to about 100 gsm, and more preferably about 30 gsm to about 50 gsm. The prefilter 424 may be configured such that a user may apply pressure through the pouch walls to squeeze out any liquid absorbed by the first layer 425. The second layer 423 is optional. In embodiments where the prefilter 424 does not include the second layer 423, the first layer 425 may be directly sealed to the pouch wall 412.

The protective panel 420 may be formed from a suitable microperforated film and sealed to the pouch wall 412 via a peripheral seal. In an embodiment, the protective panel 420 may be configured and sized slightly larger than the prefilter 423 to cover and seal around the prefilter 423. In other embodiments, the protective panel may be configured to cover about 1/5 to about 2/3 of an upper portion of the ostomy pouch, preferably, about 1/4 to about 1/2 of an upper portion of the ostomy pouch. The microperforated film may be formed from a suitable polymeric material configured for heat sealing to the pouch wall 412. In an embodiment, the protective panel 420 may be formed from a copolymer comprising about 8% ethylene-vinyl acetate (EVA). The protective panel 420 may have a thickness of about 0.5 mil to about 10 mil, preferably about 1 mil to about 5 mil.

The protective panel 420 may comprise microperforations in a portion, in more than one portion or throughout the whole area of the protective panel 420. In the embodiment of FIG. 8, the protective panel 420 may include microperforations 430 only in a lower portion of the protective panel 420. In such an embodiment, gas collected in the ostomy pouch 400 may flow through the microperforations 430 in the lower portion of the protective panel 420 and flow upward through the prefilter 424 and exit the pouch through the gas outlet opening 406 and flow through the membrane layer 404 and filtered via the filter media 418 before exiting the filter assembly 416 as shown by the arrows in FIG. 8.

The protective panel 420 may include microperforations defined by a plurality of generally circular cylindrical openings having a diameter of about 50 µm to about 500 µm, preferably about 100 µm to about 450 µm, more preferably about 150 µm to about 400 µm. In an embodiment, the protective panel 420 may include microperforations in a lower portion of the protective panel 420, wherein the microperforations have a pore-density of about 10 pores per inch (ppi) to about 500 ppi, preferably about 100 ppi to about 300 ppi. In some embodiments, the protective panel 420 may include microperforations of various sizes, various patterns, various shapes, and/or in selected portions of the protective panel 420.

FIG. 14 shows an ostomy pouch comprising a protective panel 520 according to an embodiment, wherein the protective panel 520 includes a first set of microperforations 530 in a lower portion of the protective panel 520 and a second set of microperforations 532 arranged above the first set of microperforations 530. The first set of microperforations 530 may be defined by a plurality of openings having a larger diameter than those of the second set of microperforations 532. For example, the first set of microperforations 530 may be defined by a plurality of generally circular cylindrical openings having a diameter of about 250 µm to about 500 µm, preferably about 300 µm to about 400 µm, and more preferably about 350 µm to about 380 µm. The second set of microperforations 532 may be defined by generally circular cylindrical openings having a diameter of about 50 µm to about 300 µm, preferably about 100 µm to about 250 µm, and more preferably about 125µm to about 175 µm.

In an embodiment, the protective panel 520 may be formed from a copolymer film containing about 8% EVA and having a thickness of about 2.1 mil and may comprise microperforations, wherein the microperforations include the first set of microperforations 530 defined by a plurality of openings having a diameter of about 380 µm arranged in two rows and the second set of microperforations 532 defined by a plurality of opening having a diameter of about 150 µm arranged in 24 rows, wherein the microperforations have a pore-density of about 100 ppi. The protective panel 520 may be configured for coarse particulate blocking and heat seal to pouch walls along its periphery. In some embodiments, the protective panel 520 may be provided with slits or openings proximate a lower periphery to allow any liquid accumulated between the protective panel and the pouch wall to flow down.

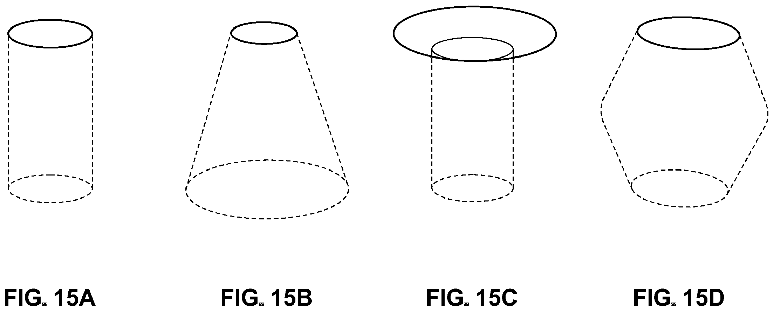

The protective panel may include microperforations formed by openings having various 3D shapes penetrating through the thickness the protective panel, such as the 3D shaped shown in FIGS. 15A-15D.

It is understood that the relative directions described above, e.g, "upward," "downward," "upper," "lower," "above," "below," are used for illustrative purposes only and may change depending on an orientation of the ostomy pouch and/or the patient. Accordingly, this terminology is non-limiting in nature. In addition, it is understood that one or more various features of an embodiment above may be used in, combined with, or replace other features of a different embodiment described herein.

In the present disclosure, the words "a" or "an" are to be taken to include both the singular and the plural. Conversely, any reference to plural items shall, where appropriate, include the singular.

From the foregoing it will be observed that numerous modifications and variations can be effectuated without departing from the scope of the concepts of the present invention. It is to be understood that no limitation with respect to the specific embodiments illustrated is intended or should be inferred. The disclosure is intended to cover by the appended claims all such modifications as fall within the scope of the claims.

## Claims

1. An ostomy pouch (10, 100, 400) comprising:
a body-side wall and a distal-side wall joined at an outer periphery (14) and defining an interior volume comprising a collection area;
an inlet for receiving ostomy effluent;
an outlet (406) for egress of gas collected in the collection area;
a filter assembly (16, 116, 216, 316, 416) covering the outlet; and
a multi-stage filter protection (217) configured to protect the filter assembly, wherein the filter assembly is attached to an outer surface or an inner surface of one of the body-side wall and the distal-side wall such that the membrane layer covers the outlet,
wherein the multi-stage filter protection comprises a prefilter (24, 124, 224, 324, 424) and a protective panel (20, 220, 320, 420, 520) covering the prefilter,
**characterised in that** the filter assembly comprises a membrane layer (104, 204, 304, 404), a backing layer (202, 402), and a filter media (18, 218, 318, 418) arranged therebetween, wherein the membrane layer is formed from a spunbond-meltblown-spunbond polypropylene (SMS PP) nonwoven.

2. The ostomy pouch (10, 100, 400) of claim 1, wherein the filter assembly (16, 116, 216, 316, 416) is attached to an outer surface of the distal-side wall and the prefilter (24, 124, 224, 324, 424) and the protective panel (20, 220, 320, 420, 520) are attached to an inner surface of the distal-side wall.

3. The ostomy pouch (10, 100, 400) of claim 1, wherein the filter assembly (16, 116, 216, 316, 416), the prefilter (24, 124, 224, 324, 424), and the protective layer (20, 220, 320, 420, 520) are attached to an inner surface of the distal-side wall.

4. The ostomy pouch (10, 100, 400) of any of claims 1-3, wherein the protective panel (20, 220, 320, 420, 520) is formed from a microperforated film, an embossed film, or a microperforated embossed film.

5. The ostomy pouch (10, 100, 400) of claim 4, wherein the protective panel (20, 220, 320, 420, 520) is formed from a microperforated film comprising a plurality of openings having a diameter of about 100 µm to 500 µm, wherein the plurality of openings are arranged to provide a pore-density of about 9.8 pores per cm to 118.2 pores per cm (25 ppi to about 300 ppi), wherein the protective panel is sealed to the distal-side wall and/or the body-side wall along an outer peripheral seal.

6. The ostomy pouch (10, 100, 400) of any of claims 4-5, wherein the protective panel (20, 220, 320, 420, 520) is formed from a microperforated film comprising a first set of microperforations comprising a plurality of openings having a diameter of about 300 µm to about 500 µm, and a second set of microperforations comprising a plurality of openings having a diameter of about 50 µm to about 200 µm, wherein the first set of microperforations are arranged proximate a lower periphery of the protective panel and the second set of the microperforations are arranged above the first set of microperforations closer to the prefilter (24, 124, 224, 324, 424).

7. The ostomy pouch (10, 100, 400) of any of claims 5-6, wherein the protective panel (20, 220, 320, 420, 520) is sealed to the distal-side wall along a lower periphery to provide a horizontal seal (22), wherein at least a portion of the protective panel proximate the filter assembly is free of openings.

8. The ostomy pouch (10, 100, 400) of claim 7, wherein the horizontal seal (22) is a discontinuous heat seal or the protective panel (20, 220, 320, 420, 520) includes at least one slit or opening proximate the lower periphery configured to allow liquid accumulated between the protective panel and the distal-side wall to flow down into the collection area.

9. The ostomy pouch (10, 100, 400) of any of claims 1-8, wherein the prefilter (24, 124, 224, 324, 424) includes a first layer (425) formed from a reticulated foam or an open cell foam.

10. The ostomy pouch (10, 100, 400) of claim 9, wherein the prefilter (24, 124, 224, 324, 424) further comprises a second layer (423), wherein the first layer (425) is laminated to the second layer and the prefilter is attached to the distal-wall by heat sealing the second layer to an inner surface of the distal-wall, wherein the second layer is formed from a polyester nonwoven or a SMS PP nonwoven.

11. The ostomy pouch (10, 100, 400) of claim 1, wherein the backing layer (202, 402) is formed from a low density polyethylene film.

12. The ostomy pouch (10, 100, 400) of claim 10, wherein the filter media (18, 218, 318, 418) is formed from an activated carbon impregnated foam, wherein the activated carbon impregnated foam is hydrophobic.

13. The ostomy pouch (10, 100, 400) of claim 1, wherein the SMS PP nonwoven has a basis weight of about 40 gsm to about 80 gsm.

14. The ostomy pouch (10, 100, 400) of any of claims 1-13, wherein the filter assembly (16, 116, 216, 316, 416) is configured to provide a radial gas flow path through the filter media (18, 218, 318, 418), wherein filter assembly is configured to direct the gas egressing through the outlet to flow through the membrane layer and radially flow through the filter media and exit the filter assembly through at least one gas outlet (308) provided proximate an outer periphery of the filter assembly.

15. The ostomy pouch (10, 100, 400) of any of claims 1-14, wherein the filter assembly (16, 116, 216, 316, 416) and the multi-stage protection are configured and arranged to allow the gas collected in the collection area to flow through microperforations (430) provided in the protective panel and flow through the prefilter (24, 124, 224, 324, 424) and exit the ostomy pouch through the outlet and flow through the membrane layer and flow through the filter media radially before exiting the filter assembly.

## Patentansprüche

1. Stomabeutel (10, 100, 400), umfassend:
eine körperseitige Wand und eine distalseitige Wand, die an einem Außenumfang (14) verbunden sind und ein Innenvolumen definieren, das einen Sammelbereich umfasst;
einen Einlass zur Aufnahme von Stomaausfluss;
einen Auslass (406) zum Austritt von in dem Sammelbereich gesammeltem Gas;
eine den Auslass abdeckende Filterbaugruppe (16, 116, 216, 316, 416); und
einen mehrstufigen Filterschutz (217), der dazu konfiguriert ist, die Filterbaugruppe zu schützen, wobei die Filterbaugruppe an einer Außenfläche oder einer Innenfläche von einer der körperseitigen Wand und der distalseitigen Wand derart befestigt ist, dass die Membranschicht den Auslass abdeckt, wobei der mehrstufige Filterschutz einen Vorfilter (24, 124, 224, 324, 424) und eine den Vorfilter abdeckende Schutzplatte (20, 220, 320, 420, 520) umfasst, **dadurch gekennzeichnet, dass** die Filterbaugruppe eine Membranschicht (104, 204, 304, 404), eine Trägerschicht (202, 402) und ein dazwischen angeordnetes Filtermedium (18, 218, 318, 418) umfasst, wobei die Membranschicht aus einem Spunbond-Meltblown-Spunbond-Polypropylen(SMS PP)-Vlies ausgebildet ist.

2. Stomabeutel (10, 100, 400) nach Anspruch 1, wobei die Filterbaugruppe (16, 116, 216, 316, 416) an einer Außenfläche der distalseitigen Wand befestigt ist und der Vorfilter (24, 124, 224, 324, 424) und die Schutzplatte (20, 220, 320, 420, 520) an einer Innenfläche der distalseitigen Wand befestigt sind.

3. Stomabeutel (10, 100, 400) nach Anspruch 1, wobei die Filterbaugruppe (16, 116, 216, 316, 416), der Vorfilter (24, 124, 224, 324, 424) und die Schutzschicht (20, 220, 320, 420, 520) an einer Innenfläche der distalseitigen Wand befestigt sind.

4. Stomabeutel (10, 100, 400) nach einem der Ansprüche 1-3, wobei die Schutzplatte (20, 220, 320, 420, 520) aus einer mikroperforierten Folie, einer Prägefolie oder einer mikroperforierten Prägefolie ausgebildet ist.

5. Stomabeutel (10, 100, 400) nach Anspruch 4, wobei die Schutzplatte (20, 220, 320, 420, 520) aus einer mikroperforierten Folie ausgebildet ist, die eine Vielzahl von Öffnungen mit einem Durchmesser von etwa 100 µm bis 500 µm umfasst, wobei die Vielzahl von Öffnungen angeordnet ist, um eine Porendichte von etwa 9,8 Poren pro cm bis 118,2 Poren pro cm (25 ppi bis etwa 300 ppi) bereitzustellen, wobei die Schutzplatte entlang einer äußeren Umfangsdichtung mit der distalseitigen Wand und/oder der körperseitigen Wand abgedichtet ist.

6. Stomabeutel (10, 100, 400) nach einem der Ansprüche 4-5, wobei die Schutzplatte (20, 220, 320, 420, 520) aus einer mikroperforierten Folie ausgebildet ist, umfassend einen ersten Satz von Mikroperforationen, der eine Vielzahl von Öffnungen mit einem Durchmesser von etwa 300 µm bis etwa 500 µm umfasst, und einen zweiten Satz von Mikroperforationen, der eine Vielzahl von Öffnungen mit einem Durchmesser von etwa 50 µm bis etwa 200 µm umfasst, wobei der erste Satz von Mikroperforationen in der Nähe eines unteren Umfangs der Schutzplatte angeordnet ist und der zweite Satz von Mikroperforationen über dem ersten Satz von Mikroperforationen näher an dem Vorfilter (24, 124, 224, 324, 424) angeordnet ist.

7. Stomabeutel (10, 100, 400) nach einem der Ansprüche 5-6, wobei die Schutzplatte (20, 220, 320, 420, 520) entlang eines unteren Umfangs an der distalseitigen Wand abgedichtet ist, um eine horizontale Dichtung (22) bereitzustellen, wobei mindestens ein Abschnitt der Schutzplatte in der Nähe der Filterbaugruppe frei von Öffnungen ist.

8. Stomabeutel (10, 100, 400) nach Anspruch 7, wobei die horizontale Dichtung (22) eine diskontinuierliche Heißsiegelung ist oder die Schutzplatte (20, 220, 320, 420, 520) mindestens einen Schlitz oder eine Öffnung in der Nähe des unteren Umfangs beinhaltet, der bzw. die dazu konfiguriert ist, zwischen der Schutzplatte und der distalseitigen Wand ansammelte Flüssigkeit nach unten in den Sammelbereich strömen zu lassen.

9. Stomabeutel (10, 100, 400) nach einem der Ansprüche 1-8, wobei der Vorfilter (24, 124, 224, 324, 424) eine erste Schicht (425) beinhaltet, die aus einem netzartigen Schaum oder einem offenzelligen Schaum ausgebildet ist.

10. Stomabeutel (10, 100, 400) nach Anspruch 9, wobei der Vorfilter (24, 124, 224, 324, 424) ferner eine zweite Schicht (423) umfasst, wobei die erste Schicht (425) auf die zweite Schicht laminiert ist und der Vorfilter durch Heißsiegeln der zweiten Schicht an einer Innenfläche der distalen Wand an der distalen Wand befestigt ist, wobei die zweite Schicht aus einem Polyestervlies oder einem SMS-PP-Vlies ausgebildet ist.

11. Stomabeutel (10, 100, 400) nach Anspruch 1, wobei die Trägerschicht (202, 402) aus einer Polyethylenfolie niedriger Dichte ausgebildet ist.

12. Stomabeutel (10, 100, 400) nach Anspruch 10, wobei das Filtermedium (18, 218, 318, 418) aus einem mit Aktivkohle imprägnierten Schaum ausgebildet ist, wobei der mit Aktivkohle imprägnierte Schaum hydrophob ist.

13. Stomabeutel (10, 100, 400) nach Anspruch 1, wobei das SMS-PP-Vlies ein Flächengewicht von etwa 40 g/m² bis etwa 80 g/m³ aufweist.

14. Stomabeutel (10, 100, 400) nach einem der Ansprüche 1-13, wobei die Filterbaugruppe (16, 116, 216, 316, 416) dazu konfiguriert ist, einen radialen Gasströmungsweg durch das Filtermedium (18, 218, 318, 418) bereitzustellen, wobei die Filterbaugruppe dazu konfiguriert ist, das durch den Auslass austretende Gas zu leiten, um durch die Membranschicht zu strömen und radial durch das Filtermedium zu strömen und durch mindestens einen Gasauslass (308), der in der Nähe eines Außenumfangs der Filterbaugruppe bereitgestellt ist, aus der Filterbaugruppe auszutreten.

15. Stomabeutel (10, 100, 400) nach einem der Ansprüche 1-14, wobei die Filterbaugruppe (16, 116, 216, 316, 416) und der mehrstufige Schutz konfiguriert und angeordnet sind, um dem in dem Sammelbereich gesammelten Gas zu ermöglichen, durch Mikroperforationen (430) zu strömen, die in der Schutzplatte bereitgestellt sind, und durch den Vorfilter (24, 124, 224, 324, 424) zu strömen und durch den Auslass aus dem Stomabeutel auszutreten und durch die Membranschicht zu strömen und vor dem Austritt aus der Filterbaugruppe radial durch das Filtermedium zu strömen.

## Revendications

1. Poche de stomie (10, 100, 400) comprenant :
une paroi côté corps et une paroi côté distal réunies au niveau d'une périphérie externe (14) et définissant un volume intérieur comprenant une zone de collecte ;
une entrée de réception d'effluent de stomie ;
une sortie (406) pour la sortie du gaz collecté dans la zone de collecte ;
un ensemble filtre (16, 116, 216, 316, 416) recouvrant la sortie ; et
une protection de filtre à étages multiples (217) configurée pour protéger l'ensemble filtre, dans laquelle l'ensemble filtre est fixé à une surface externe ou à une surface interne de l'une parmi la paroi côté corps et la paroi côté distal de sorte que la couche de membrane recouvre la sortie, dans laquelle la protection de filtre à étages multiples comprend un préfiltre (24, 124, 224, 324, 424) et un panneau de protection (20, 220, 320, 420, 520) recouvrant le préfiltre, **caractérisée en ce que** l'ensemble filtre comprend une couche de membrane (104, 204, 304, 404), une couche de support (202, 402) et un milieu filtrant (18, 218, 318, 418) agencé entre celles-ci, dans laquelle la couche de membrane est formée à partir d'un non-tissé de polypropylène de spunbond-meltblown-spunbond (SMS PP).

2. Poche de stomie (10, 100, 400) selon la revendication 1, dans laquelle l'ensemble filtre (16, 116, 216, 316, 416) est fixé sur une surface externe de la paroi côté distal et le préfiltre (24, 124, 224, 324, 424) et le panneau de protection (20, 220, 320, 420, 520) sont fixés sur une surface interne de la paroi côté distal.

3. Poche de stomie (10, 100, 400) selon la revendication 1, dans laquelle l'ensemble filtre (16, 116, 216, 316, 416), le préfiltre (24, 124, 224, 324, 424) et la couche de protection (20, 220, 320, 420, 520) sont fixés sur une surface interne de la paroi côté distal.

4. Poche de stomie (10, 100, 400) selon l'une quelconque des revendications 1 à 3, dans laquelle le panneau de protection (20, 220, 320, 420, 520) est formé à partir d'un film microperforé, d'un film gaufré ou d'un film gaufré microperforé.

5. Poche de stomie (10, 100, 400) selon la revendication 4, dans laquelle le panneau de protection (20, 220, 320, 420, 520) est formé à partir d'un film microperforé comprenant une pluralité d'ouvertures ayant un diamètre d'environ 100 µm à 500 µm, dans laquelle la pluralité d'ouvertures sont agencées pour fournir une densité de pores d'environ 9,8 pores par cm à 118,2 pores par cm (25 ppi à environ 300 ppi), dans laquelle le panneau de protection est scellé à la paroi côté distal et/ou à la paroi côté corps le long d'un joint périphérique externe.

6. Poche de stomie (10, 100, 400) selon l'une quelconque des revendications 4 et 5, dans laquelle le panneau de protection (20, 220, 320, 420, 520) est formé à partir d'un film microperforé comprenant un premier ensemble de microperforations comprenant une pluralité d'ouvertures ayant un diamètre d'environ 300 µm à environ 500 µm, et un second ensemble de microperforations comprenant une pluralité d'ouvertures ayant un diamètre d'environ 50 µm à environ 200 µm, dans laquelle le premier ensemble de microperforations sont agencées à proximité une périphérie inférieure du panneau de protection et le second ensemble de microperforations sont agencées au-dessus du premier ensemble de microperforations, plus près du préfiltre (24, 124, 224, 324, 424).

7. Poche de stomie (10, 100, 400) selon l'une quelconque des revendications 5 et 6, dans laquelle le panneau de protection (20, 220, 320, 420, 520) est scellé à la paroi côté distal le long d'une périphérie inférieure pour fournir un joint horizontal (22), dans laquelle au moins une partie du panneau de protection à proximité de l'ensemble filtre est exempte d'ouvertures.

8. Poche de stomie (10, 100, 400) selon la revendication 7, dans laquelle le joint horizontal (22) est un joint thermique discontinu ou le panneau de protection (20, 220, 320, 420, 520) comporte au moins une fente ou ouverture à proximité de la périphérie inférieure configurée pour permettre au liquide accumulé entre le panneau de protection et la paroi côté distal de s'écouler vers le bas dans la zone de collecte.

9. Poche de stomie (10, 100, 400) selon l'une quelconque des revendications 1 à 8, dans laquelle le préfiltre (24, 124, 224, 324, 424) comporte une première couche (425) formée d'une mousse réticulée ou d'une mousse à cellules ouvertes.

10. Poche de stomie (10, 100, 400) selon la revendication 9, dans laquelle le préfiltre (24, 124, 224, 324, 424) comprend également une seconde couche (423), dans laquelle la première couche (425) est stratifiée à la seconde couche et le préfiltre est fixé à la paroi distale par thermoscellage de la seconde couche sur une surface interne de la paroi distale, dans laquelle la seconde couche est formée d'un non-tissé de polyester ou d'un non-tissé de SMS PP.

11. Poche de stomie (10, 100, 400) selon la revendication 1, dans laquelle la couche de support (202, 402) est formée d'un film de polyéthylène basse densité.

12. Poche de stomie (10, 100, 400) selon la revendication 10, dans laquelle le milieu filtrant (18, 218, 318, 418) est formé d'une mousse imprégnée de charbon actif, dans laquelle la mousse imprégnée de charbon actif est hydrophobe.

13. Poche de stomie (10, 100, 400) selon la revendication 1, dans laquelle le non-tissé de SMS PP a un poids de base compris entre environ 40 gsm et environ 80 gsm.

14. Poche de stomie (10, 100, 400) selon l'une quelconque des revendications 1 à 13, dans laquelle l'ensemble filtre (16, 116, 216, 316, 416) est configuré pour fournir un trajet d'écoulement de gaz radial à travers le milieu filtrant (18, 218, 318, 418), dans laquelle l'ensemble filtre est configuré pour diriger le gaz sortant à travers la sortie pour s'écouler à travers la couche de membrane et s'écouler radialement à travers le milieu filtrant et sortir de l'ensemble filtre à travers au moins une sortie de gaz (308) prévue à proximité d'une périphérie externe de l'ensemble filtre.

15. Poche de stomie (10, 100, 400) selon l'une quelconque des revendications 1 à 14, dans laquelle l'ensemble filtre (16, 116, 216, 316, 416) et la protection à étages multiples sont configurés et agencés pour permettre au gaz collecté dans la zone de collecte de s'écouler à travers des microperforations (430) prévues dans le panneau de protection et de s'écouler à travers le préfiltre (24, 124, 224, 324, 424) et de sortir de la poche de stomie à travers la sortie et de s'écouler à travers la couche de membrane et de s'écouler à travers le milieu filtrant radialement avant de sortir de l'ensemble filtre.
